# EUROPEAN PATENT SPECIFICATION

(11) **EP 2 528 543 B1**
(45) Date of publication and mention of the grant of the patent: **05.10.2016**
(21) Application number: 11701141.1
(22) Date of filing: 27.01.2011
(51) Int. Cl.: A61F 2/08, B22C 3/00, B28B 7/36

(54) **METHOD AND DEVICE FOR MODELLING TENDINOUS TISSUE INTO A DESIRED SHAPE**
VERFAHREN UND VORRICHTUNG ZUR MODELLIERUNG VON SEHNENGEWEBE IN EINE GEWÜNSCHTE FORM
PROCÉDÉ ET DISPOSITIF POUR MODELER UN TISSU TENDINEUX SOUS UNE FORME DÉSIRÉE

(30) Priority: 28.01.2010 EP 10151932
(43) Date of publication of application: 05.12.2012
(73) Proprietor: Universität Zürich, 8006 Zürich (CH)
(72) Inventor: SNEDEKER, Jess G., 8008 Zürich (CH); MEYER, Dominik Christoph, 8032 Zürich (CH); FARSHAD TABRIZI, Mazda, 8044 Gockhausen (CH)
(74) Representative: Habermann, Hruschka & Schnabel
(86) International application number: PCT/EP2011/051171
(87) International publication number: WO 2011/092262

(56) References cited:
- EP-A1- 0 520 177
- EP-A1- 2 172 231
- WO-A1-03/022319
- WO-A1-2004/067052
- WO-A2-2009/004351
- US-A- 4 048 380
- US-A- 5 247 005
- US-A- 5 458 973
- US-A1- 2004 028 875

## Description

The present invention relates to methods and devices for the modelling tendinous tissue into a desired shape. The shaped tissue produced by the methods of the present invention is useful in any field relating to biological tissue, particularly in the field of medicine, most preferred in surgery, for intraoperative use or pre- or peri-operative preparation of the tissue. The handling of soft tissue is a common task in surgery. Soft tissue, such as a tendon may be surgically connected to various types of other soft tissues, such as a tendon to tendon suture or a vascular repair, closure of the abdominal cavity or the like. In orthopaedic surgery, however, the fixation to bone is very common.

In the fixation of soft tissue such as a cruciate ligament transplant, there are several unsolved problems: Usually a bone hole is drilled and a transplant is pulled into the tunnel. Fixation of the transplant should allow the healing of a transplant to bone with following optimal properties: i) close contact to seal the transplant against the joint space and fluid, ii) circumferential contact to allow for circumferential bone ingrowth, iii) press-fit of tendon to bone, iv) no micro-motion between tendon and bone, v) appropriate local homeostasis with preferably homogeneous adaptation of the soft tissue against the surrounding bone, vi) no implants such as interference screws which create and potentially leave a bone tunnel or hole in the bone where not needed, vii) anatomical shape and size of the transplant. If those requirements are not met, following problems may arise: 1) widening and progressive enlargement of the bone tunnel, with effects called such as "windshield wiper" or "bungee cord" effects, 2) only unilateral attachment to bone, 3) necrosis of the tendon end, 4) problems during revision surgery, 5) non-anatomical movement of joint and transplant, 6) formation of cysts between bone and transplant or within the bone or transplant.

To avoid loose contact of cruciate ligament transplants in the bone tunnel, several techniques have been developed, which, however, all have particular disadvantages: Fixation with an interference screw: in this technique, a transplant bundle is introduced into the bone tunnel and an interference screw (alternatively also a dowel, splint, wedge or the like) may be inserted into the canal, holding the transplant in the tunnel against the opposite tunnel wall in an non-homogeneous pattern and sealing the transplant in the tunnel against joint fluid resulting in a weak mechanical hold and no circumferential contact of the transplant with the bone and a wide bone tunnel. After healing of the transplant, the screw remains in place and after resorption of the screw, usually a hole of the size of the screw, a non-anatomical position persists. If a re-operation is needed, then this hole has to be filled with bone graft and healing has to occur for typically 3-4 months before a revision ligament transplantation can be performed. To avoid the problem of the interference screw in the bone tunnel, also a so-called hybrid fixation may be performed: hold for the transplant is ensured by a cortical fixation device such as a flipping device (Flip-Tack), retrograde bone buttress or the like. A bone chip is loosened next to the tunnel and a screw is inserted such that it compresses the chip against the transplant. In this manner, a circumferential bone contact may be achieved, however, with only moderate mechanical hold. The problem of a screw next to the tunnel in a non-anatomical position, however, remains. Furthermore, the step is technically demanding and in case of a double-bundle ligament repair, there is not enough space for two screws.

Cortical fixation: there are several types of fixation techniques, which allow for a fixation of the bundle by fixation of the suture which is holding the transplant at the surface of the bone, for example at a cross-plate, button or screw. The problem of this technique is that the transplant construct is relatively long and elastic and the so-called bungee-effect may occur. Metaphyseal fixation: in this method the ligament is inserted into the tibial and/or femoral tunnel, and polymeric or metallic cross-inserted implants are inserted perpendicularly through the tunnel, passing through the implant, however, resulting in no tight contact of bone and transplant and a hybrid fixation is not possible because the cross-pins would be compromised.

A device according to the preamble of claims 1 and 4 is known from the document EP-A-0 520 177.

An object of the present invention is to provide methods and devices for the modeling of tendinous tissue to a desired shape. The inventors have surprisingly found that compression of tendinous tissue does not lead to breakage of the tissue, but the tissue loses up to about 50% of the water content without elastic recoil and therefore becomes smaller and easier to handle. Particularly such compressed tendinous tissue can be more easily inserted into a hole (such as a drill hole in bone) or a groove or other tight space. When fluid is added to the tissue it regains its former shape. The expansion leads to tight contact with the bone. Hence the method and devices of the present invention are used to produce a tissue transplant with an improved fit in tight space, which makes in situ compression unnecessary.

In particular, according to a first aspect, the present invention provides a method for modelling tendinous tissue into a desired shape according to claim 1.

The time of compression depends on the size and diameter of the tendinous tissue, the amount of the desired volume reduction, enhancing measures and the like. Compression time is preferably from a fraction of a second to several days, more preferred from 1 second to one day, most preferred from 1 second to 30 minutes.

The amount of pressure depends on the size and diameter of the tendinous tissue, the amount of the desired volume reduction, enhancing measures and the like. It is preferably of from 0.1 N mm⁻² to 10.5 N mm⁻², more preferred from 1 N mm⁻² to 1000 N mm⁻², most preferred from 1 N mm⁻², to 100 N mm⁻².

The amount of pressure, time, size of modified tissue and type of enhancing measures are interrelated and influence each other.

The compression may be repeated at least once, preferably from 1 to 1000000 times, most preferred 1 to 20 times.

The tissue can be placed in one moulding template only or in several moulding templates with different shapes consecutively.

Optionally, in the course of the compression step, compounds and devices can be forced into the tissue by the pressurization. This could include any biomaterial including minerals (e.g. calcium, phosphate, magnesium), or surgical devices like screws, stents, sutures and the like.

Pharmacological substances could also be incorporated under pressure (e.g. growth factors, bone inducing factors, stimulative factors, antibiotics), powders, stents, stents with pharmacological adjuncts, microspheres containing pharmacological adjuncts, cells (e.g. embryonic, stem or stem-like or pluripotent cells), and fluids (e.g. physiological solutions, water, blood, serum).

In particular within the above meaning of forcing compounds or devices into the tissue by pressurization, a coating, e.g. a bioactive (such as osteogenic), is applied to, impregnated into, or attached to the tissue by pressurization. The coating contains or is made of a mineraloid substance. The form of the coating substance can be stent-like, helical, spiral, cylindrical or alike. These covers or coatings, respectively, for example having the above forms or structures may be permeable or impermeable to body fluids, cells or tissue, i.e. the stent or comparable structure may be made or be in the form of a porous or non-porous mesh, textile or sheath. It may also be equipped with members reaching into the transplant or around each of the tendinous tissue, in particular tendon bundles forming a transplant such as an ACL graft. A coating with mineraloid substances such as tri-calcium phosphate (TCP), preferably granulate or sintered TCP, together with or without a stent-like device or a stent-like device consisting fully or partially of or containing a mineraloid substance such as TCP or a similar substance or combination of such substances or compounds is attached to or impregnated into the tissue by the method of according to the invention.

Optionally, the tissue can be physically modified before, during and/or after pressurization (once or repetitive), e.g. by application of heat and/or cold, application of electric current, application of vibration (e.g. ultrasonic vibration), application of radiation (e.g. gamma-radiation, UV), application of tensile or compressive force or application of magnetic fields. To model the tissue or as an adjunct to the pressurization (before/during and/or after pressurization), chemical methods can be used, e.g. chemical dry out of the tissue by e.g. hyperosmolar solutions, chemical modification of the tissue (e.g. oxidation, or convective dehydration using heated/dry air.

During step (b) of the method according to the invention the tissue usually loses water. It is therefore preferred that the mould contains means for discharging water leaking from the tissue. Typical water discharging means is/are one or more holes in the mould.

The method of the invention makes it also feasible to provide the surface of the tendinous tissue with a predetermined texture, in particular by selecting a mould having an adequate inner surface structure that is imprinted on the tissue during step (b).

According to a further aspect, the present invention provides a device for modelling tendinous tissue into a desired shape according to claim 4.

Preferably, the device of the invention further comprises a sensor for measuring the pressure applied on at least a part of the tendinous tissue.

According to preferred embodiments of the invention, the mould of the device is formed by a two-partite template, which two parts of the template are inserted into pressurisation means for applying pressure onto the tendinous tissue inserted into the template in a guided direction.

Different means for pressurising the tendinous tissue present in the template are envisaged: For example, the means for applying pressure onto the tendinous tissue may be formed by vice jaws (examples see Figs. 1, 3, 4), which may be driven, e.g. by a screw system (either manually (see Figs. 1, 3) or by use of an electric motor) or by a(n) (electro-)magnetic (see Fig. 4), pneumatic or hydraulic system.

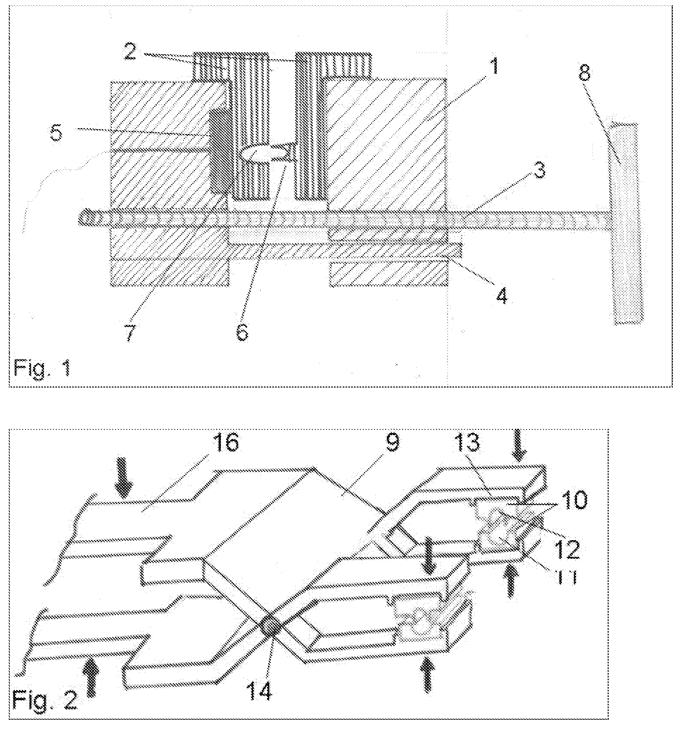

The means for applying pressure onto the tendinous tissue may also be formed by a forceps-like device having one or more receptacles each receiving a template (Fig. 2).

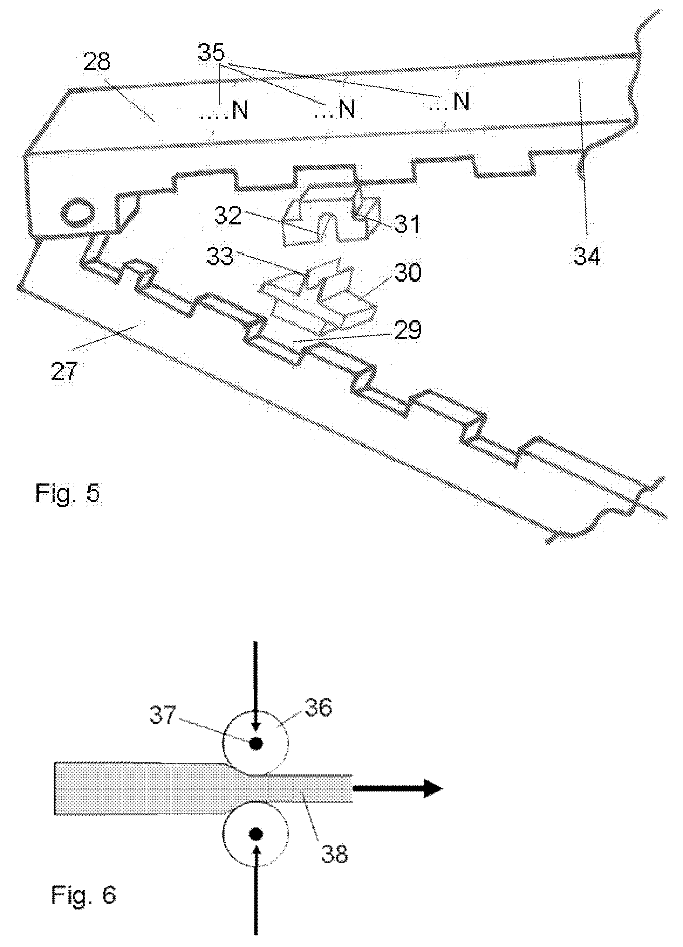

A further embodiment is a lever device having one or more receptacles each receiving a template between two lever arms (Fig. 5).

Further embodiments of the invention are devices comprising two opposing and rotating cylinders between the tendinous tissue can be drawn through (Fig. 6).

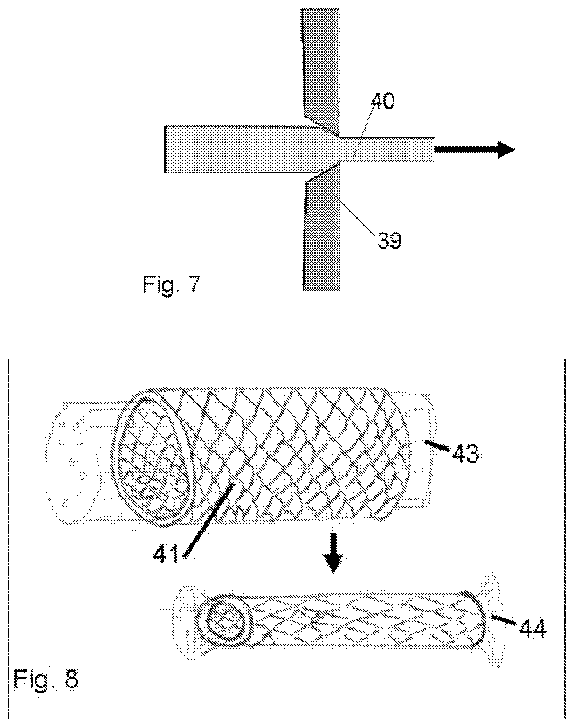

The device may also comprise a template comprising two parts through which the tendinous tissue can be drawn (Fig. 7).

According to a further embodiment, the mould is a stent for receiving the tendinous tissue, which stent has a diameter which can be decreased at least in a region of said stent when said tendinous tissue or at least a part thereof is inserted there into.

The present invention is also directed to the use of the inventive device for modelling tendinous tissue into a desired shape. Thus, a method for modelling tendinous tissue into a desired shape (in particular as generally defined above) using the inventive device is also subject matter of the present invention.

As mentioned before, it is preferred that the mould comprises means such as one or more holes for discharging water leaking from the tissue when compressed in the mould.

Also disclosed are certain moulds adapted for, or comprised in, respectively the device according to the invention. Such moulds are foreseen to apply a coating on the tendinous tissue wherein the coating is equipped with members sucht that, after pressurization, these members reach into the tissue and/or reach out of the tendindous tissue, in particular tendon bundles in order to provide a high friction force when the tendon is inserted into the hole of a bone, for example during ACL reconstruction.

Thus, the mould of the invention comprises a lining material on its inner surface (or at least a part of the inner surface) which lining contains on at least a part thereof members that reach at least partially into the tendinous tissue such that after pressurization of the tendinous tissue in the mould the tissues (or at least a part thereof) is provided with a corresponding coating.

The above-described members are realized by making the lining material of a mineraloid substance or compound. A preferred mineraloid substance in the context of the present invention is tricalcium phosphate (TCTP), particularly preferred granulate or sintered TCP.

As mentioned before with respect to the coating applied to the tendinous tissue, the lining material preferably has structure such that it can be pulled over the tendinous tissue, preferably it has a stent-like, helical, sprial or cylindrical structure or it is a combination of such structures. Particular preferred embodiments of the present invention are stent-like structures. Preferably, the lining material has a form such that a coating on the tendinous tissue results that does not cover the complete tissue or a complete part of the tissue, but rather covers only certain regions of the tissue in a predetermined pattern having interspaced parts in which no coating is present. Thus, according to preferred embodiments of the present invention, the lining material may be in the forms of a porous or non-porous (preferably porous) mesh, textile or sheath.

Not part of the present invention but also disclosed are certain objects that are adapted to serve as a lining material in the mould as defined herein, i.e. they can be inserted into the mould, in particular a mould of the device as disclosed herein. This object is preferably made of or contains a mineraloid substance or compound, preferably, as already outlined above, TCP, in particular TCP granules or TCP sinter material. Preferred structures and form of such objects have been already disclosed above.

Particularly preferred objects adapted as lining materials for the mould of the invention are therefore stent-like or other structures as described elsewhere herein, for example a mesh, textile or sheath, or a combination thereof, of a biocompatible material containing TCP, preferably in the form of granulate TCP or sintered TCP.

Especially preferred moulds/objects as defined above are described in more detail in the Example section and the Figures.

The present invention is also directed to the inventive device as described herein above modified with moulds as defined above.

The present invention is further described in detail with reference to the accompanying drawings in which:
Figure 1 shows a device having a template (2) with a preferentially cylindrical pressurization shape (7/6) being inserted into a pressurization device (1), where pressure can be applied in a guided (4) direction, preferably by a screw-system (3) with a lever arm (8). The force is monitorized by an integrated sensor (5).
Figure 2 shows a device having one or two templates (10) with a preferentially cylindrical pressurization shape (11/12) being inserted into a pressurization device (2), wherein pressure can be applied in a guided (14) direction, preferably by a lever arm (16). The tissue can be shaped at one or two ends.

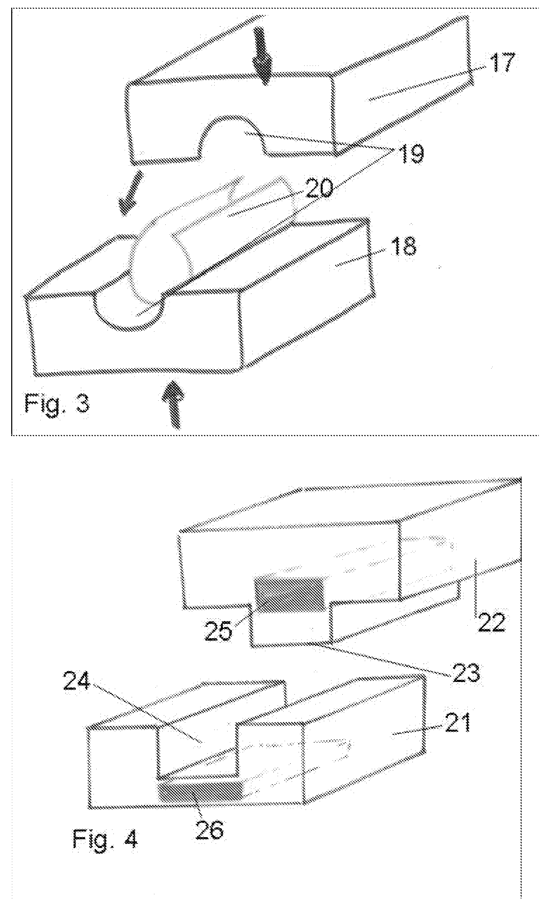

Figure 3 shows a device having a template (20) with a preferentially cylindrical pressurization shape being inserted into a device (17/18) with preferentially cylindrical shape (19). Any pressurization device can be used to compress the device (17/18), and the force can be monitorized, if desired.
Figure 4 shows a device (21/22) having a rectangular pressurization design (23/24), where the pressurization force derives from implanted magnets of any strength (25/26).
Figure 5 shows a device having a template (30/31) with a preferentially cylindrical pressurization shape (32/33) being inserted into a pressurization device (27/28/29), wherein pressure can be applied in a guided direction, preferably by a lever arm (34). The force is preset, by insertion of the templates according to the ranked visualization (35).
Figure 6 shows a device wherein the tissue (38) is drawn trough two opposing cylinders which rotate around a centre (37) and wherein angular force can be applied.
Figure 7 shows a device wherein the tissue (40) is drawn trough a preformed template (39).
Figure 8 shows a stent like device (41) surrounding the tissue (43). With modification of the shape of the device, in particular by reducing the diameter of the stent, the tissue is forced to adapting the shape (44) of the (tightened) stent.

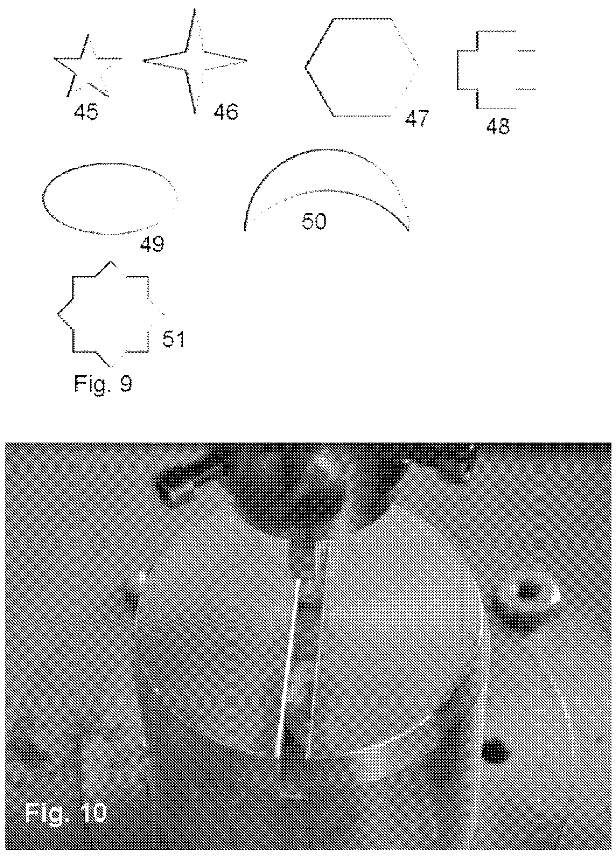

Figure 9 shows various cross sections of shapes of the tissue that can be achieved by the inventive method and devices.
Figure 10 shows a device allowing pressurization of the tendon with an electromechanical testing machine (Zwick GMBH, Germany).

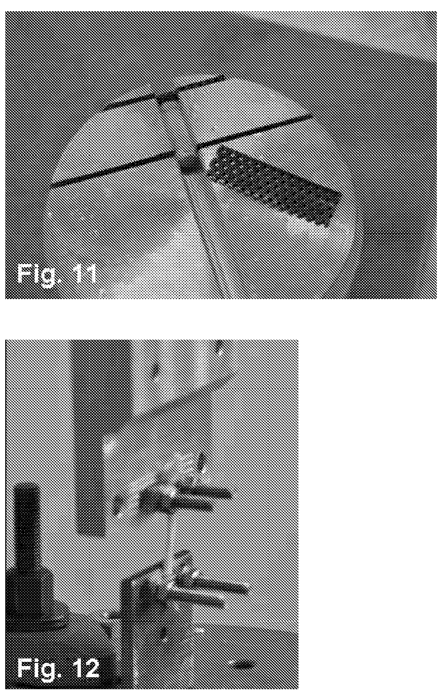

Figure 11 shows a modified device as shown in Fig. 10 wherein borders were constructed using porous metal to prevent expansion of the tendon in longitudinal direction and to allow contact with fluids.
Figure 12 shows a device developed for fixation of the tendon strips.

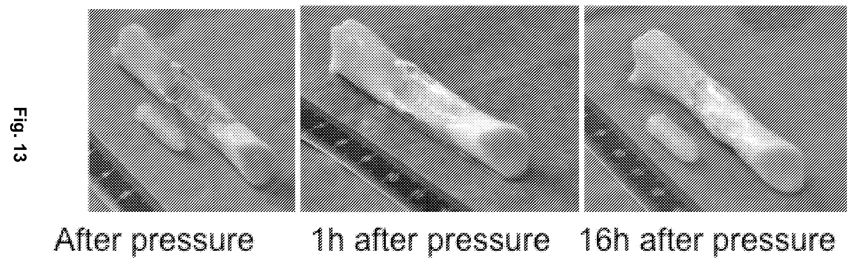

Figure 13 shows photographs for documenting the feasibility of modelling tendons reversibly. A tendon was compressed with an interference screw. Photographs were taken immediately after, 1 hour and 16 hours after pressurization.

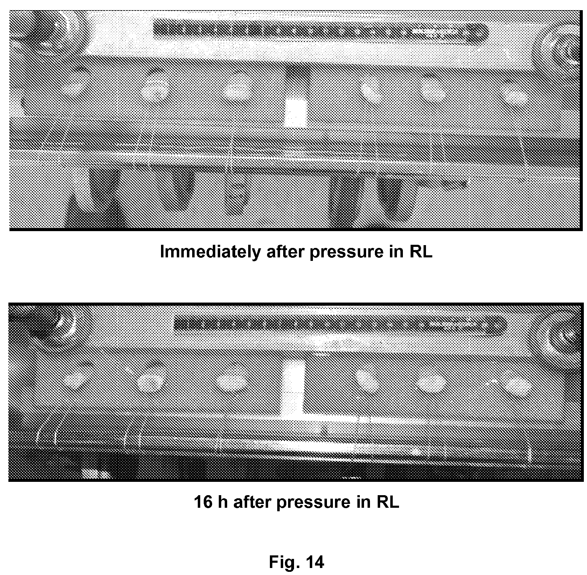

Figure 14 shows a photographs of 3 compressed tendons (right side in each photograph) and 3 non-compressed control tendons (left side in each photograph) after they were put into predrilled holes in sawbone and exposed to Ringer lactate for 16 h.

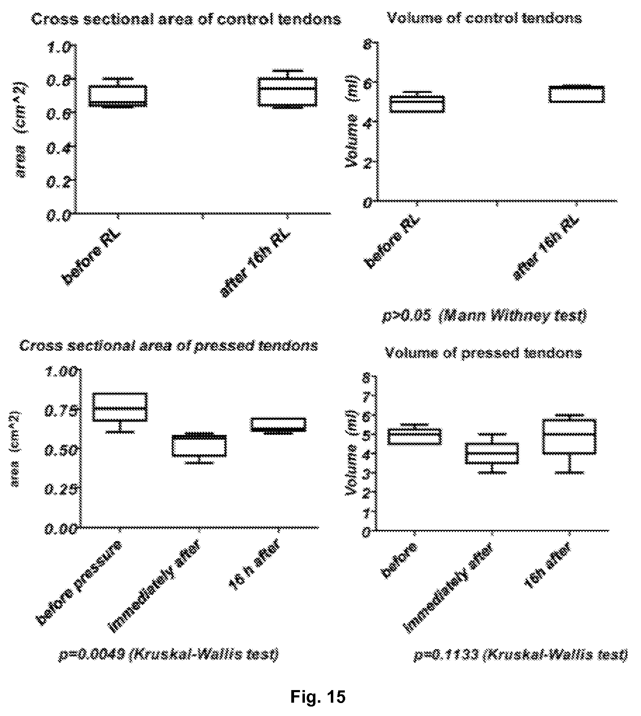

Figure 15 shows the behavior of a tendon undergoing pressurization. The cross section of the control tendons of group II remained unchanged from before (0.68 ± 0.06 cm²) to (0.72 ±0.08 cm²) after exposition to RL for 16 h (p=0.60). However, for tendons undergoing pressurization, the cross sectional area changed from 0.76 ± 0.09 cm² before to 0.52 ± 0.07 cm² (p=0.0117) and restored to 0.64 ± 0.04 cm², which was not a significant difference to the beginning (p=0.093).

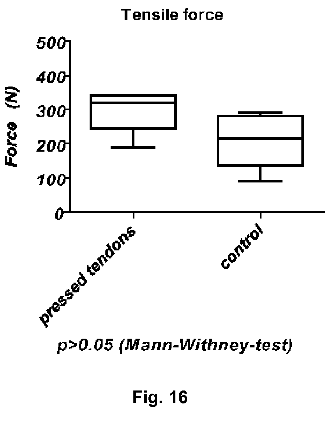

Figure 16 shows the tensile force capacity of tendon strips undergoing pressurization (298 ± 62 N) in comparison to the control group (210 ± 79 N) (p=0.084).

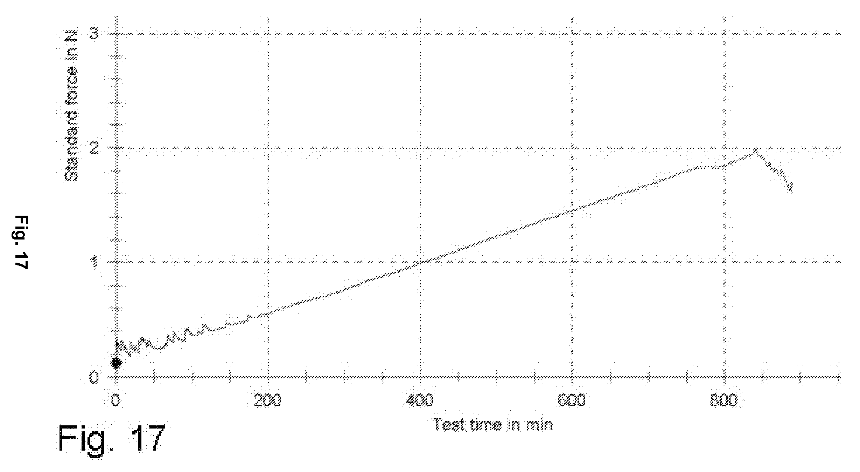

Figure 17 shows the results of expansion force measurements. The qualitative measurement for the expansion force on one tendon revealed a progressive force with a peak of 2 N.

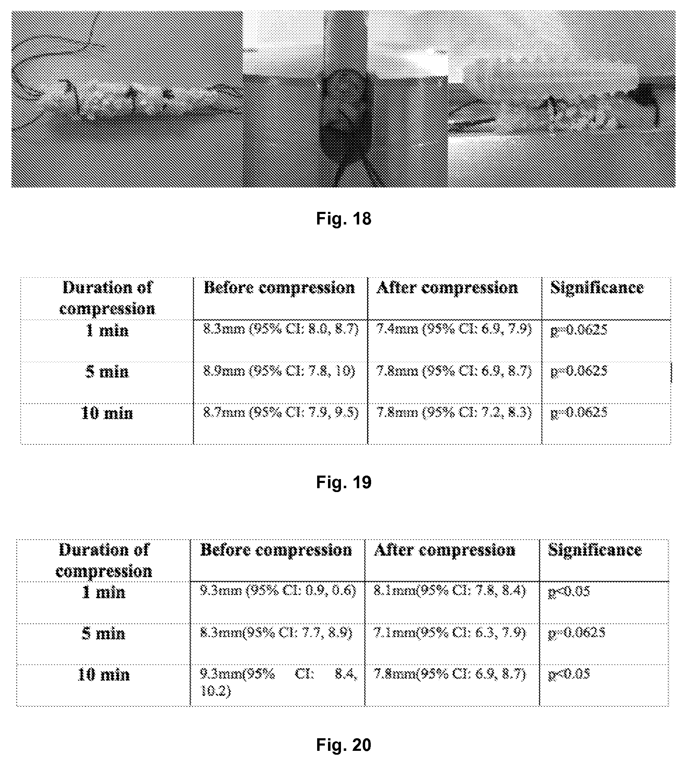

Figure 18 shows a preferred embodiment of a coating of tendinous tissue with TCP granula and pressurization of the graft with a screw-formed template for embossing the form of the screw into the coated tendinous tissue.
Figure 19 shows a table of diameter measurements on tendon bundle grafts demonstrating diameter reduction of bundled grafts for different times of single compressive hold of 6000N.
Figure 20 shows a table of diameter measurements on tendon bundle grafts demonstrating diameter reduction of bundled grafts for different times of creeping compressive hold of 6020 N.

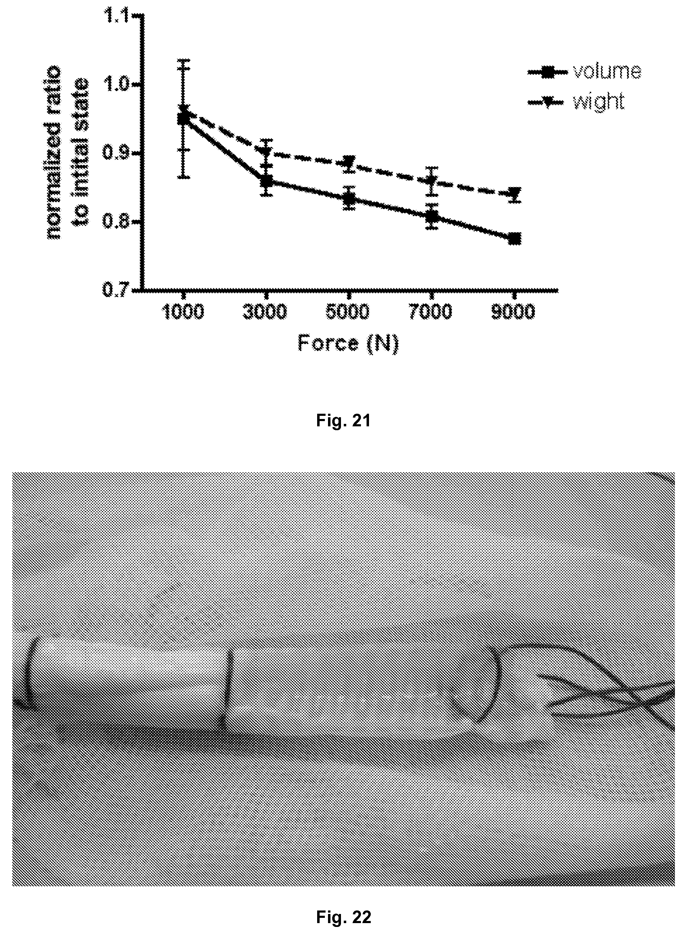

Figure 21 shows that the average initial bundle diameters were reduced after 1, 5 and 10 minutes of single force compression, respectively.
Figure 22 shows a tendon bundle which has undergone screw embossing by pressurization of a screw-formed template into the tissue.

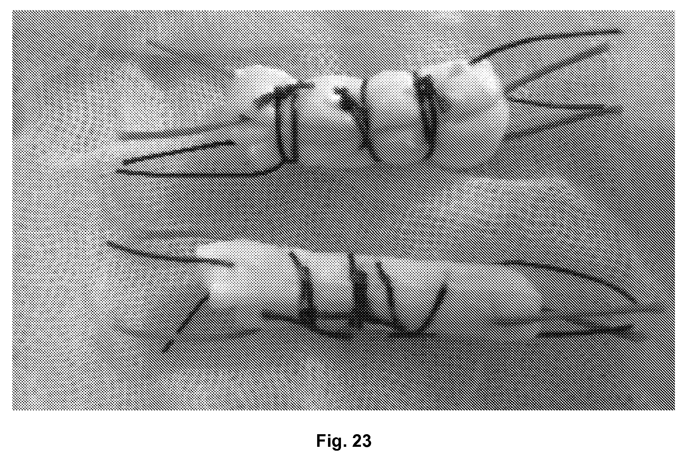

Figure 23 shows an uncompressed tendinous bundle (top) and a compressed bundle (bottom) after carrying out the method according to the invention which demonstrates the more clear definition of form and increase of length as well as decrease in volume and diameter after pressurization.

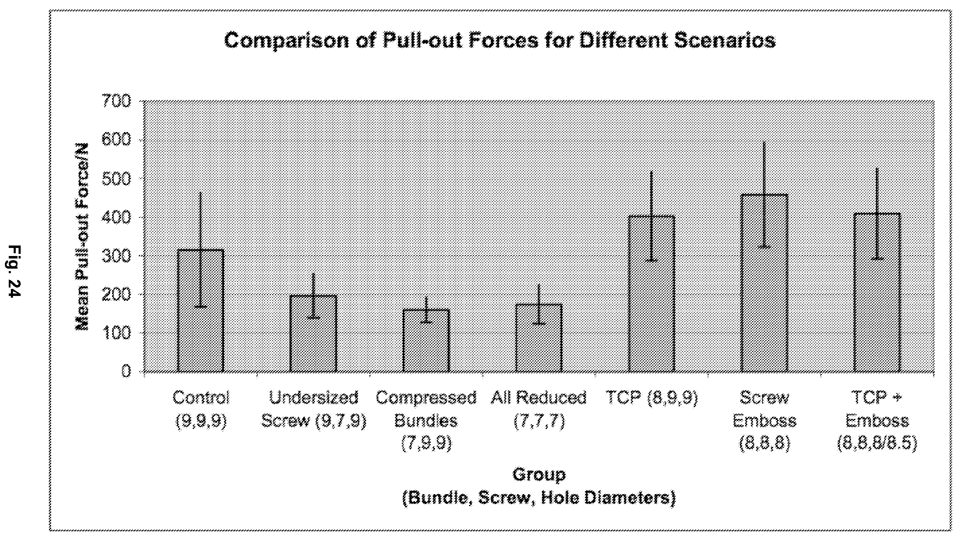

Figure 24 shows a comparison of pullout forces of different bundles sized with different screws fixed into different hole sizes with and without previous tissue pressurization and with or without TCP coating and/or screw embossing.

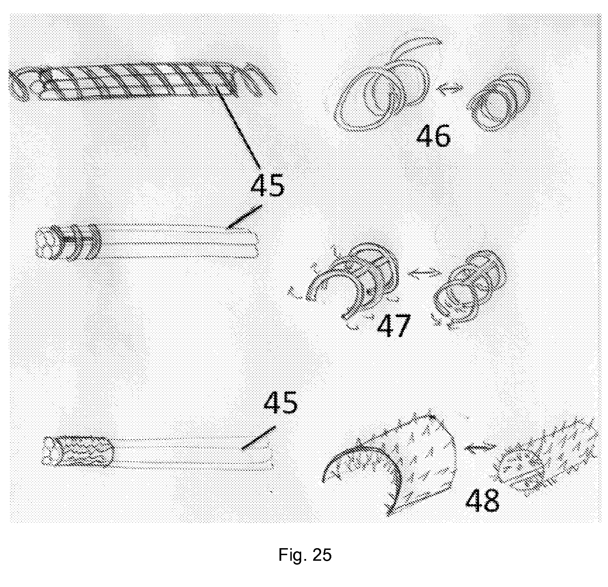

Figure 25 shows different possibilities of coating the tissue (45) with any kind of material. The design of the coat could be helical (46) or with open circles (47) or like a sheet which can cover the tissue, for example partially or substantially completely (48).

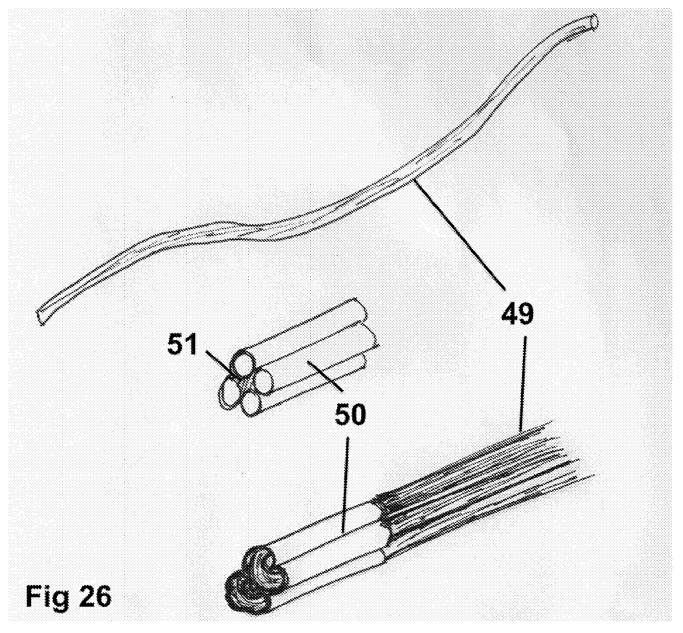

Figure 26 shows a further coating embodimentusing any kind of material covering the tendinous tissue (49) and serving as a guide before folding (50) and allowing insertion of any material between the arms and/or having any material attached to the coating (51).

The device of the present invention may comprise two parts: Part A ((1 in Fig.1, 9 in Fig. 2, 17/18 in Fig. 3, 21/22 in Fig. 4, 2728 in Fig 5) or (36/37 in Fig 6 and 39 in Fig 7)) is used to apply mechanical force, most preferred quantified/monitored by an integrated sensor (5 in Fig. 1). The mechanical force can be applied by manpower, electricity, magnetic fields (26/25 in Fig. 4), hydraulic or pneumatic systems. A template, Part B, (2/6/7 in Fig. 1, 10 in Fig. 2, 20 in Fig. 3, 30/31 in Fig. 5, 41 in Fig. 8) is a mould consisting of a negative form of a desired shape (e.g. round cylinder, oval, quadrangle, ribbed pipe, pyramid, cylinder, cube, threaded screw form or reversed screw form, thin strips) which allows forming the tissue to the desired shape (45-51 in Fig. 9). Part B (2 in Fig. 1, 10 in Fig. 2, 20 in Fig. 3, 30/31 in Fig. 5, 41 in Fig. 8) is produced / provided in different shapes, sizes, length and colours and can be connected to Part A (1 in Fig. 1, 9 in Fig. 2, 17/18 in Fig. 3, 21/22 in Fig. 4, 27/28 in Fig. 5) or (36/37 in Fig. 6 and 39 in Fig. 7) independently of inner shape, size, length, colour or treated inner surface. The template, Part B, can be connected to other devices which can apply pressure with use of adaptors.

The components of the device can be of any suitable material, e.g. polymers, metals, wood, carbon, ceramic, biomaterials, engineered materials (e.g. polymethyl methacrylate, bone cement, silicone) or smart memory alloys. Preferably, Part B (2 in Fig. 1, 10 in Fig. 2, 20 in Fig. 3, 30/31 in Fig. 5, 41 in Fig. 8) is made of polyethylene and Part A (1 in Fig. 1, 9 in Fig. 2, 17/18 in Fig. 3, 21/22 in Fig. 4, 27/28 in Fig. 5 or 36/37 in Fig. 6 and 39 in Fig. 7) is made of a metal.

The device or components of the device can be reusable or disposable. Furthermore, the device or its compounds is/are preferably made of material(s) which can be sterilized, in particular in case of reusable components or in case of a reusable device, respectively.

Optionally, the device can be prepared or manufactured to contain devices or substances which in the course of the pressurization are forced into the tissue. Such devices or substances are e.g. minerals, hormones, pharmacologics, sutures, anchors, cables, powders, magnets, stents, bone, muscle, osteoinductive substances, vitamins, cells, microspheres, biologic tissue, screws, soft tissues, osmotic substances, synthetic tissues, fluids, polymers, metals, natural materials (e.g. wood), carbon, ceramic, biomaterials, engineered materials (e.g. polymethyl methacrylate, bone cement, silicone, etc.), smart memory alloys and combinations thereof.

According to a preferred embodiment, the present invention provides a method and device for shaping a tendon implant to be used in cruciate ligament repair. The method comprises the steps of:
(a) a tendon which may have been removed from the semitendinosis muscle or the patellar/quadriceps tendon is placed into the mould
(b) mechanical pressure is applied;
(c) the compressed tissue is removed from the mould; and
(d) the diameter and/or size of the compressed tissue is measured in order to know the size and/or shape of the hole to be drilled into the bone (tibial and femoral) in the course of the cruciate ligament repair operation. According to a preferred embodiment, the present invention provides a device for shaping an implant to be used in cruciate ligament repair. The device allows for concentric, homogeneous compression of the tendon transplant. Through the specific design, water is pressed out of the tendon. This can reduce the volume by approximately 30% and the diameter by roughly 1-2 mm. This improves the fit of the transplant in the tunnel and makes in situ canal compression unnecessary. At the same time, the surprising, unexpected and beneficial effect of partial lengthening (1-50%, in the case of cruciate ligaments preferred 5-15%) of the pressured tissue occurs. With relaxation of the lengthened state, for example a cruciate ligament transplant will shrink back to its original diameter and length, giving additional tension and thereby improved stability to the reconstructed joint. The device comprises Part B (2 in Fig. 1, 10 in Fig. 2, 20 in Fig. 3, 30/31 in Fig. 5, 41 in Fig. 8) which is disposable and is preferably made of an engineered material such as polyethylene. Most preferred, the inner shape of the Part B is cylindrical (7/8 in Fig. 1, 11/12 in Fig. 2, 20 in Fig. 3, 32/33 in Fig. 5). Most preferred, Part A (1 in Fig. 1, 9 in Fig. 2, 17/18 in Fig. 3, 21/22 in Fig. 4, 27/28 in Fig. 5 or 36/37 in Fig. 6 and 39 in Fig. 7) is a device wherein compression can be applied while monitoring the force by a sensor (5 in Fig. 1) or by manual palpation (16 in Fig. 2, 34 in Fig. 5) or visualization (35 in Fig. 5), and wherein the compression is controlled in direction by a guide (4 in Fig. 1, 14 in Fig. 2). Most preferred this is achieved by a system involving a screw (3 in Fig. 1) with a lever arm (8 in Fig. 1), which can be turned to compress two opposing plates. Alternatively, the tissue (38 in Fig. 6, 40 in Fig. 7) can be pushed or pulled through a device or die (36 in Fig. 6, 39 in Fig. 7), where additionally the device could rotate around a centre (37 in Fig. 6) and compressive force could be applied to the device. Most preferred, all parts of the device according to the present invention are sterizable.

The unexpected and unique feature of the invention is that the soft tissue (in this case tendon) does not react elastically to the pressure, but undergoes a temporary plastic deformation and the tissue can be handled (and transplanted for example). Therefore, a thinner canal can be drilled than without pressurization and the tendon fits into the hole. Unexpectedly, this does not only occur in diameter, but the tendon is temporarily longer, i.e. pre-stretched from its dimension prior to pressurization. This effect may be regarded as an indirect stretching of the tendon. After insertion of the transplant, the water re-enters the tendon tissue and the tissue tends to regain its original dimensions/volume within about 24 h. Thereafter, a soft expansive pressure promotes constant contact of tendon and bone, thus promoting the healing into the bone tunnel. In the same process, the tendon also becomes slightly shorter, accordingly providing a desirable tension on the reconstruction. The amount of pre-stretching is far greater than possible with simple pulling on the tendon ends.

The present invention is further illustrated by the following non-limiting examples.

### EXAMPLES

### Example 1: Proof of concept; study with calf (bovine) Achilles tendons

### Material and Methods:

### Experimental setup and basic behavior of the tendon undergoing pressurization:

Achilles tendons from calf were harvested, dissected from adjunctive tissue and wrapped in gauze soaked with Ringer lactate (RL). For preparation of the experiments, the tendons were thawed from frozen 24 h before the experiments at room temperature and cut uniformly to a length of 5 cm and 2.5 cm respectively. The volume, weight and diameter of 10 tendons were measured and documented. Groups of 5 tendons each were assigned to a group I and II, respectively. A device was designed (Fig. 10) to allow pressurization of the tendon with an electromechanical testing machine (Zwick 1456, Zwick GMBH, Germany). In group I, tendons underwent 10 cycles of pressure with 10 kN with pressurization time of 2 s at the peak pressure of 10 kN. Tendons in group II underwent no pressurization. All tendons were exposed to RL for 16 hours. Thereafter, the volume, weight and diameter of 10 tendons were measured and documented.

### Expansion force measurement:

One additional tendon was cut to 2.5 cm and underwent the same procedure for pressurization as described above with a 2.5 cm pressurization head. However, after 10 cycles, the sensor was changed from (20 kN sensor to a 50 N sensor) and positioned to slightly touch the surface of the tendon. Further, the borders were constructed using porous metal to prevent expansion of the tendon in longitudinal direction (Fig.11) and allow contact with RL. The tendon was then exposed to RL for 12 hours, and the expansion force was measured.

### Tensile strength measurement:

One tendon was cut in half, and each half was further cut in 5 equal strips. 5 strips were assigned to a group III and IV, respectively. Tendon strips in group III underwent pressurization with a 2.5 cm head with 10 kN for 10 cycles with a waiting time of 2s at a peak force of 10kN. A devise was developed for fixation of the tendon strips (Fig. 12). All tendons underwent tensile strength measurement. The maximal tensile strength before failure was documented.

### Reversibility of shaping:

To qualitatively demonstrate feasibility of modelling tendons reversibly, one tendon was pressed with an interference screw (20 x 7 mm, Linvatec), which was filled with bone cement to prevent failure of the screw (Palacos R+G 2x20, Haraeus Medical GmbH, 61273 Wherheim, Germany), with 10 cycles of 10 kN peak force and waiting time of 2 s on that force. Photodocumentation was performed immediately after, 1 hour and 16 hours after pressurization (Fig. 13).

### Behavior of the tendon in the bone tunnel:

3 tendons were randomly assigned to a group V, where pressure was applied with 10 cycles of 10 kN. Another group of 3 tendons (group VI) were not exposed to pressure. Size 2 Fiberwire sutures were used to grasp the tendons and to pull through the predrilled holes (diameter of 8 mm in group V and 12 mm in group VI, respectively) in a cellular rigid polyurethane foam (Sawbones, 12.5 pcf., Sweden). The tendons and sawbones were put in RL for 16 hours and photodocumentation was performed at time 0 and after 16 hours (Fig. 14).

### Statistical analysis:

Data of the experiments for basic behavior of the tendon undergoing pressurization and from the experiments investigating the tensile force capacity were analyzed statistically using Kruskal Wallis test for intergroup comparison for 3 time points or Mann Whitney test for 2 time points, respectively.

### Results:

### Behavior of the tendon undergoing pressurization:

The cross section of the control tendons of group II remained unchanged (Fig. 15) from before (0.68 ± 0.06 cm2) to (0.72 ± 0.08 cm2) after exposition to RL for 16 h (p=0.60). However, for tendons undergoing pressurization, the cross sectional area changed from 0.76 ± 0.09 cm2 before to 0.52 ± 0.07 cm2 (p=0.0117) and restored to 0.64 ± 0.04 cm2, which was not a significant difference to the beginning (p=0.093).

The tensile strength was not significantly different (Fig. 16) in the tendon strips undergoing pressurization (298 ± 62 N), if compared to the control group (210 ± 79 N) (p=0.084).

### Expansion force measurement:

The qualitative measurement for the expansion force on one tendon revealed a progressive force with a peak of 2 N (Fig 17).

### Example 2: Biomechanical characterization of tissue pressurization - Experiments on bundled calf and/or sheep flexor digitorum tendon grafts:

### Introduction:

Tunnel size enlargement and graft relaxation are the two major contributors to failure after anterior cruciate ligamament (ACL) reconstruction with hamstring grafts. The aim was to characterize the biomechanical behavior of the graft under compression and find the most effective way to reduce volume of the graft.

### Material and Methods:

Flexor digitorum tendons of calf and extensor digitorum tendons of adult sheep were identified to be suitable as ACL grafts substitutes for human hamstring tendons in vitro. The effect of different compression forces on dimensions and weight of the grafts were determined. Further, different strain rates (1 mm/min vs 10mm/min), compression modes (steady compression vs. creep) and different compression durations (1, 5, 10min) were tested to identify the most effective combination to reduce graft size by preserving its macroscopic structure.

### Volume reduction in relation to the compressive force

Experiments for characterization of the effect of compression on volume were performed on 13 sheep extensor digitorum tendons, which were bundled to provide a bundle having a diameter of 8 mm and a length of 30 mm. To determine the diametric change due to compression at different peak loads in steps of 2000N, sample weight, length, height and width were measured before and after compression. The measured diameters were then compared to a theoretical compressed diameter, calculated from the weight change due to water exuding from the tendon. This was done by determining the volumetric change of the tendon from the mass of the water loss, giving a theoretical compressed volume. From the measured length of the compressed graft, the compressed area and, therefore, diameter could be calculated. This comparison was done to see if weight loss can be an indicator of diametric reduction, as the weight loss would be a more repeatable measurement, compared to graft diameter. 2 or 3 grafts were tested at compression force level time for reproducibility.

### Strain rate

Relaxation tests to determine an appropriate strain rate were performed at 10mm/min and 1 mm/min, to determine how stable the tendon was under the different rates of loading. The trade-off here was between the time taken to reach peak load (and therefore the main time constraint, if the hold time was short) and consistency of the compressed tendon. 6 grafts were tested at the lower strain rate for reporducibility.

### Single compression tests

Load was then increased to 6000N at 1 mm/min and held at a fixed displacement for the desired time (1, 5 or 10 minutes), after which samples were unloaded. 5 grafts were tested at each hold time for reproducibility.

### Effective creep tests

The compression force was increasedup to 6020N at 1 mm/min and holding for 1 second before decreasing to 5990N for 1 second. Cycles between these two forces were performed until the desired duration (1, 5 or 10 minutes) had been achieved, after which grafts were unloaded completely. Approximately 20, 75 and 150 cycles corresponded to 1, 5 and 10 minutes, respectively, of load-controlled loading. 5 grafts were tested at each hold time for reproducibilitypeatability.

### Results:

### Volume reduction in relation to the compressive force

The volume decreased with increasing compression force (Figure 21) and reached a plateau at about 6kN (75% of initial volume). The decrease of volume was associated with a decrease in weight (Figure 21) and a decrease in area and diameter both being parameters determining the volume.

### Single Compression Tests

The average initial bundle diameters were reduced similarly after 1, 5 and 10 minutes of compression (Figure 19). Diametric reductions were found to be reasonably constant for relaxation periods between 1 and 10 minutes (p=0.25).

### Creep Tests

The average initial diameter of the bundles used in creep testing were reduced to a relevant amount (Figure 20). There seemed to be no significant gains in terms of diametric reduction for a creep period of 10 minutes compared to 1 minute (p=0.5).

### Conclusion:

Compression reduces the dimensions of the ACL graft and could contribute to overcoming problems of tunnel size enlargement and graft relaxation leading to failure after ACL reconstruction. The present in vitro experiments suggest a preferred preconditioning of the graft by creeping compression with 6kN at a strain rate of 1 mm/min.

### Example 3: Biomechanical performance of compressed and surface enhanced ACL grafts - Experiments on bundled calf flexor digitorum tendon grafts:

### Introduction:

Tunnel widening is the unavoidable consequence of the interference screw fixation technique for anterior cruciate ligament (ACL) reconstruction caused by insertion of a screw in the same sized bone tunnel filled with a graft of also the same size. The viscoelastic behavior of the graft results in immediate relaxation after screw fixation. Both, tunnel widening and graft relaxation result in inferior hold. Grafts pre-conditioned through mechanical compression and surface enhancement trough TCP or screw embossing to decrease the viscous behavior could allow preventing tunnel enlargement during screw insertion by simultaneously providing superior pullout strength.

### Material and methods:

Fresh flexor digitorum tendons of the calf were used to create bundles with a diameter of 9 mm and inserted into sawbone tunnels. 7 groups were formed to compare pullout strength and bone damage in constructs of compressed tendons (group II: 7 mm) some with TCP coating (group V, 8 mm) or screw embossing (group VI, 8 mm) or both (group VII) and inserted into a 9 mm (groups I, II, III, V), 8 mm (groups VI, VII) or a 7 mm hole (group IV) and fixed by a 7 mm (groups II, IV), 8 mm(groups VI, VII) or a 9 mm screw (groups I, III, V).

### Results:

Compression allowed to use a smaller screw (7mm) and a smaller bone tunnel (7mm) (group IV), however, significant lower pullout strength of 174 N (95%CI: 97,250) compared to the control 315N (204, 426)). TCP-coated (402 N (243,561)), as well as screw embossed grafts (458 N (302,614)) and combination of both (409N, 95%CI:274,543) showed substantiallyhigher pullout strengths, if compared to the prior art technique. Tunnel enlargement was high in group I, III, IV, and V, and low in group II, VI, and VII (Figure 24).

### Conclusion:

The present embodiment of the method of preconditioning the graft by mechanical compression with use if TCP or screw embossing according to the invention allows reduction of bone tunnel width and decreases bone damage by simultaneously providing superior pullout strength.

### Example 4: Method of transplant shaping by the example of a semitendinosus cruciate ligament autograft transplant, quadrupled

(a) The clinical procedure is first performed using techniques known in the art to harvest a cruciate ligament graft, up to the point of harvesting the graft for reconstruction of the cruciate ligament.
(b) The semitendinosus (or other graft tendon) is stripped from the muscle bed and released from the insertion on the tibia. Then both ends are grasped for example by sutures or other suitable implants and the graft is folded to a quadrupled strand.
(c) At the folded ends, a suture loop holds the implant, which is for example connected to a cortical fixation device. The transplant is then inserted into the pressurization apparatus of the invention and compressed with 10 kN for three seconds, ten times. Through this measure the collagen loses up to about 50% of the water content without elastic recoil.
(d) The diameter of the pressured transplant is measured and a corresponding hole drilled into femur or tibia as it is performed by the current state of the art techniques. These holes may also be expanded to fit the transplant.
(e) The transplant is inserted into the bone preferentially within 20 minutes and fixated in the common, preferably cortical manner. Through the pressurization technique of the invention, however, no hybrid fixation or fixation with a bone wedge is needed. And through the following shortening of the transplant, a soft tension is developing within the construct, tightening the repair and new ligament.
(f) The procedure is then continued and concluded using commonly used methods to complete reconstruction of the cruciate ligament.

### Example 5: Method of tissue shaping by the example of biceps tenodesis

(a) Biceps tenodesis is performed by using common methods of shoulder surgery, up to the point of gathering the proximal end of the biceps tendon for refixation.
(b) After the tendon is released from the origin of the superior glenoidal rim, the end is grasped, for example by sutures or other suitable implant device.
(c) The tendon end is then inserted into the pressurization apparatus of the invention and compressed with 10 kN for three seconds, ten times. Through this measure the collagen looses up to about 50% of the water content without elastic recoil.
(d) The diameter of the pressured tendon is measured and a corresponding hole drilled into humerus. The hole may also be expanded to fit the transplant.
(e) The transplant is inserted into the bone, preferentially within 20 minutes and fixated with an anchor, interference screw or the like. However, the tendon will expand and fixation will be more stable than without previous pressurization.
(f) The procedure is then continued and concluded using commonly used methods for biceps tenodesis.

### Example 6: Method of tissue shaping by the example of lateral ankle stabilization

(a) The procedure is performed using common techniques to stabilize the lateral aspect of the ankle (distal fibula to talus and/or calcaneus) with the use of tendinous tissue (allo- or autograft).
(b) After surgical approach to the region, the tissue is inserted into the pressurization apparatus according to the invention and compressed with 10 kN for three seconds, ten times. Through this measure the collagen loses up to about 50% of the water content without elastic recoil.
(c) The diameter of the pressured tissue is measured and a corresponding hole drilled into distal fibula and talus and/or calcaneus. The hole may also be expanded to fit the transplant.
(d) The transplant is inserted into the bone preferentially within 20 minutes and fixated with sutures. The tendon will expand and fixation will be more stable than without previous pressurization. The shortening of the transplant with water immersion will help to uphold the soft tension on the ligaments.
(e) The procedure is then continued and concluded using common methods for ankle stabilization.

### Example 7: Method of transplant shaping of a semitendinosus cruciate ligament

### autograft transplant, quadrupled WITH the use of a tricalcium phosphate layer

(a) The clinical procedure is first performed using techniques known in the art to harvest a cruciate ligament graft, up to the point of harvesting the graft for reconstruction of the cruciate ligament.
(b) The semitendinosus (or other graft tendon) is stripped from the muscle bed and released from the insertion on the tibia. Then both ends are grasped for example by sutures or other suitable implants and the graft is folded to a quadrupled strand.
(c) At the folded ends, a suture loop holds the implant, which is for example connected to a cortical fixation device. The transplant is then inserted into the pressurization apparatus. The apparatus has been pre-equipped with a cover in the mould made of tricalcium phosphate granules (may also be bone for example), optionally held in a mesh of a bioabsorbable material such as polylactic acid, collagen, gels and the like. During the compression of the graft with 10 kN for three seconds, ten times, the granules are pressed into the tendon graft and massively increase the surface of the collagen bundle (underneath the granules). Through this measure the collagen loses up to about 50% of the water content without elastic recoil. The recoil is further reduced through the stent-like optional layer.
(d) The diameter of the pressurized transplant is measured and a corresponding hole drilled into femur or tibia as it is performed by the current state of the art techniques. These holes may also be expanded to fit the transplant.
(e) The transplant is inserted into the bone preferentially within 20 minutes and fixated in the usual, preferably cortical manner. Through the pressurization technique of the invention, however, no hybrid fixation or fixation with a bone wedge is needed. And through the following shortening of the transplant, a soft tension is developing within the construct, tightening the repair and new ligament.
(f) The procedure is then continued and concluded using commonly used methods to complete reconstruction of the cruciate ligament.

## Claims

1. A method for modelling tendinous tissue into a desired shape comprising the steps of:
(a) placing at least a part of the tendinous tissue into a mould representing the negative form of the desired shape; and
(b) applying mechanical pressure on at least the part of the tendinous tissue in the mould to compress the tendinous tissue;
wherein at least a part of the tissue is provided with a coating during step (b)
**characterized in that**
the coating contains or is made of a mineraloid substance

2. The method of claim 1 wherein the mineraloid substance is tricalcium phosphate (TCP).

3. The method of claim 2 wherein the TCP is granulate or sintered TCP.

4. A device for modelling tendinous tissue into a desired shape comprising
- a mould for receiving at least a part of the tendinous tissue, which mould is made of a biocompatible material and represents the negative form of the desired shape of at least a part of the tendinous tissue; and
- means for applying pressure on at least a part of the tendinous tissue placed into the mould;
the mould comprising a lining applied at least to a part of the inner surface of the mould for providing a coating on at least a part of a tendinous tissue wherein the lining contains members reaching into the tendinous tissue;
**characterized in that**
the lining contains or is made of a mineraloid substance.

5. The device of claim 4 wherein the mineraloid substance is tricalcium phosphate (TCP).

6. The device of claim 5 wherein the TCP is granulate or sintered TCP.

## Patentansprüche

1. Verfahren zur Modellierung von Sehnengewebe in eine gewünschte Form, umfassend die Schritte:
(a) Einsetzen mindestens eine Teils des Sehnengewebes in eine Form, welche das Negativ der gewünschten Form darstellt, und
(b) Beaufschlagen des mindestens einen Teils des Sehnengewebes in der Form mit mechanischem Druck, um das Sehnengewebe zu komprimieren,
wobei mindestens ein Teil des Gewebes mit einer Beschichtung während des Schritts (b) versehen ist,
**dadurch gekennzeichnet, dass**
die Beschichtung eine mineraloide Substanz enthält oder daraus besteht.

2. Verfahren nach Anspruch 1, wobei die mineraloide Substanz Tricalciumphosphat (TCP) ist.

3. Verfahren nach Anspruch 2, wobei das TCP granuläres oder gesintertes TCP ist.

4. Vorrichtung zur Modellierung von Sehnengewebe in eine gewünschte Form, umfassend
- eine Form zur Aufnahme mindestens eines Teils des Sehnengewebes, wobei die Form aus einem biokompatiblen Material besteht und das Negativ der gewünschten Form des mindestens einen Teils des Sehnengewebes darstellt, und
- Mittel zur Beaufschlagung des mindestens einen Teils des Sehnengewebes, das sich in der Form befindet, mit einem Druck, wobei die Form eine Auskleidung umfasst, die auf mindestens einem Teil der inneren Oberfläche der Form aufgebracht ist, um auf mindestens einem Teil eines Sehnengewebes eine Beschichtung bereitzustellen, wobei die Auskleidung Elemente enthält, welche in das Sehnengewebe hineinreichen,
**dadurch gekennzeichnet, dass**
die Auskleidung eine mineraloide Substanz enthält oder daraus besteht.

5. Vorrichtung nach Anspruch 4, wobei die mineraloide Substanz Tricalciumphosphat (TCP) ist.

6. Vorrichtung nach Anspruch 5, wobei das TCP granuläres oder gesintertes TCP ist.

## Revendications

1. Méthode de modelage d'un tissu tendineux dans une forme désirée comprenant les étapes suivantes :
(a) placer au moins une partie du tissu tendineux dans un moule représentant le négatif de la forme souhaitée, et
(b) appliquer une pression mécanique sur au moins une partie du tissu tendineux se trouvant dans le moule pour compresser le tissu tendineux,
dans laquelle un revêtement est appliqué sur au moins une partie du tissue au cours de l'étape (b), **caractérisée en ce que** le revêtement contient ou est fait d'une substance minérale.

2. Méthode selon la revendication 1, **caractérisé en ce que** la substance minérale est le phosphate de tricalcium (TCP).

3. Méthode selon la revendication 2, **caractérisé en ce que** le TCP est sous forme de granulé ou fritté.

4. Dispositif de modelage d'un tissu tendineux dans une forme désirée comprenant,
- un moule de réception d'au moins une partie du tissu tendineux, ledit moule étant fabriqué à partir d'un matériau biocompatible et représentant le négatif de la forme souhaitée d'au moins une partie du tissu tendineux, et
- un moyen d'application d'une pression sur au moins une partie du tissu tendineux placé dans le moule,
le moule comprenant un matériau de revêtement appliqué sur au moins une partie de la surface intérieure du moule pour appliquer un revêtement sur au moins une partie du tissu tendineux dans lequel le matériau de revêtement contient des composantes pénétrant à l'intérieur du tissu tendineux,
**caractérisé en ce que** le matériau de revêtement contient ou est fait d'une substance minérale.

5. Dispositif selon la revendication 4, dans laquelle la substance minérale est le phosphate de tricalcium (TCP).

6. Dispositif selon la revendication 5, dans laquelle le TCP est sous forme de granulé ou fritté.
